(19) Europäisches Patentamt
European Patent Office
Office européen des brevets

(11) Numéro de publication: **0 406 050 B1**

(12) **FASCICULE DE BREVET EUROPEEN**

(45) Date de publication de fascicule du brevet: **07.09.94**

(51) Int. Cl.⁵: **C07C 37/60**, C07C 41/26

(21) Numéro de dépôt: **90401668.0**

(22) Date de dépôt: **15.06.90**

(54) **Procédé de préparation de phénols.**

(30) Priorité: **22.06.89 FR 8908582**

(43) Date de publication de la demande:
**02.01.91 Bulletin 91/01**

(45) Mention de la délivrance du brevet:
**07.09.94 Bulletin 94/36**

(84) Etats contractants désignés:
**AT BE CH DE DK ES FR GB GR IT LI LU NL SE**

(56) Documents cités:
**EP-A- 0 341 113**
**EP-A- 0 341 164**
**EP-A- 0 341 165**
**US-A- 3 709 979**

**CHEMISTRY LETTERS, no. 6, 1988, pages 953-956, The Chemical Society of Japan; E. SUZUKI et al.: "Hydroxylation of benzene with dinitrogen monoxide over H-ZSM-5 zeolite"**

(73) Titulaire: **RHONE-POULENC CHIMIE**
**25, quai Paul Doumer**
**F-92408 Courbevoie Cédex (FR)**

(72) Inventeur: **Gubelmann, Michel**
**39, Boulevard des Belges**
**F-69006 Lyon (FR)**
Inventeur: **Popa, Jean-Michel**
**2, Rue Roger Breton**
**F-93700 Drancy (FR)**
Inventeur: **Tirel, Philippe-Jean**
**40, Boulevard Kennedy**
**F-96900 Oullins (FR)**

(74) Mandataire: **Dutruc-Rosset, Marie-Claude et al**
**RHONE-POULENC CHIMIE**
**Service Brevets Chimie**
**25, Ouai Paul Doumer**
**F-92408 Courbevoie Cédex (FR)**



Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne un procédé de préparation de phénol ou de phénols substitués. Elle concerne plus particulièrement un procédé catalytique d'hydroxylation de substrats aromatiques.

L'hydroxylation du phénol, de phénols substitués ou d'éthers de phénol, par le peroxyde d'hydrogène, pour préparer des diphénols, des diphénols substitués ou des alcoxyphénols, est une réaction connue.

Le brevet français FR 2 071 464 a décrit un procédé dans lequel la réaction est catalysée par un acide fort, tel que par exemple l'acide perchlorique ou l'acide sulfurique.

Le brevet allemand n° 2 410 742 a décrit un procédé semblable au précédent, dans lequel le peroxyde d'hydrogène est mis en oeuvre sous forme de solution organique pratiquement anhydre.

Ces deux procédés sont très intéressants et le premier procédé est mis en oeuvre industriellement.

Cependant depuis quelques années, on cherche à catalyser la réaction d'hydroxylation à l'aide de solides non dissous dans le milieu, afin de simplifier leur séparation du milieu réactionnel et leur éventuel recyclage et éviter les sous-produits salins, qui se forment le plus souvent lors de l'élimination des catalyseurs acides dissous.

Ainsi, le brevet français FR 2 489 816 préconise l'emploi de silicalite de titane comme catalyseur hétérogène de l'hydroxylation de composés aromatiques par le peroxyde d'hydrogène.

En fait, cette catalyse présente de grandes difficultés de reproductibilité. En outre la fine taille des particules de catalyseur utilisées rend leur séparation du milieu réactionnel très difficile et leur recyclage problématique, alors que dans un procédé industriel, il est indispensable de pouvoir recycler un catalyseur coûteux.

Pour remédier à ce problème de la séparation du catalyseur, il a été proposé, dans la demande de brevet européen publiée sous le n° 200 260, d'utiliser des agglomérats de ces fines particules de silicalite de titane.

La difficulté de contrôler la sécurité des ateliers lors de l'utilisation de péroxyde d'hydrogène et la relative médiocrité des rendements obtenus dans l'art antérieur fait que depuis de nombreuses années, le chimiste cherche à introduire directement un groupe hydroxyle sur le noyau aromatique en l'absence de tout dérivé péroxydique. Cette chimie a longtemps échoué.

En outre, l'hydroxylation directe d'un composé aromatique, ne comportant pas de substituant ou comportant un substituant désactivant, tel que le benzène ou les halogénobenzènes n'a guère été décrite jusqu'à présent.

Une publication décrivant l'introduction directe d'un groupe hydroxyle sur un noyau benzénique consiste en un article de IWAMOTO paru dans le Journal of Physical Chemistry, 87, 6, 1983.

Cette réaction d'hydroxylation du benzène est réalisée par le protoxyde d'azote ($N_2O$) en présence d'un catalyseur à base d'un oxyde d'un métal des groupes V ou VI de la classification périodique.

L'oxyde de vanadium est l'oxyde préféré parmi les oxydes des métaux des groupes V et VI de la classification. Il est préférable d'utiliser cet oxyde en présence d'un support à base de silice en quantité pondérale comprise entre 1 et 10 % par rapport au support. Le support est constitué de préférence de silice, l'alumine provoquant majoritairement la formation d'un mélange d'oxydes de carbone.

Le procédé de IWAMOTO était intéressant, mais l'utilisation de ces catalyseurs le rendait peu attrayant pour l'industrie.

On connaît aussi la publication de E. SUZUKI (Chemistry Letters pp. 953-956, 1988) décrivant l'obtention de phénol par réaction du protoxyde d'azote sur le benzène, en présence d'une zéolite de type H-ZSM5. Cependant le rendement et la productivité en phénol restent faibles.

L'industrie est donc toujours à la recherche d'un procédé d'hydroxylation susceptible d'être appliqué à des substrats aromatiques divers et ne mettant pas en oeuvre le peroxyde d'hydrogène.

La présente invention représente une solution à ce problème. Elle a pour objet un procédé d'hydroxylation d'un composé aromatique de formule (I) :

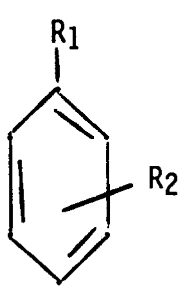

(I)

dans laquelle :

- $R_1$ représente :
  . un groupement OH,
  . un atome de brome,
  . un atome de chlore,
  . un atome de fluor,
  . un atome d'hydrogène,
  . un radical alkyle, linéaire ou ramifié, ayant 1 à 6 atomes de carbone,
  . un radical alcoxy, linéaire ou ramifié, ayant 1 à 6 atomes de carbone,
- $R_2$ représente :
  . un atome d'hydrogène,
  . un radical alkyle, linéaire ou ramifié, ayant 1 à 6 atomes de carbone,
  . un radical alcoxy, linéaire ou ramifié, ayant 1 à 6 atomes de carbone,

caractérisé en ce que l'on met en présence, à une température de 250 °C à 500 °C, le composé aromatique de formule (I), du protoxyde d'azote et une zéolite modifiée choisie parmi :

a) les zéolites modifiées de type ZSM-5 de formule générale (II) exprimée en termes de rapports d'oxydes :

$$M_2O, X_2O_n, m\ SiO_2, p\ H_2O \qquad (II)$$

dans laquelle :

- M représente un cation choisi parmi l'hydrogène et les métaux alcalins, M étant au moins en partie un atome d'hydrogène ;
- X représente Ga, Fe, B, In, Cr, Sc, Co, Ni, Be, Zn, Cu, Sb, As, V ;
- n représente 2, 3 ou 5 selon la valence de l'élément X ;
- m représente un nombre supérieur ou égal à 20 ;
- p représente un nombre de 0 à 40 ;

b) les zéolites modifiées de type ZSM-11 de formule générale (III), exprimée en termes de rapports d'oxydes :

$$M_2O, Z_2O_n, m\ SiO_2, x\ H_2O \qquad (III)$$

dans laquelle :

- M représente un cation choisi parmi l'hydrogène et les métaux alcalins, M étant au moins en partie un atome d'hydrogène ;
- Z représente Al, Ga, Fe, B, In, Cr, Sc, Co, Ni, Be, Zn, Cu, Sb, As, V ;
- n représente 2, 3 ou 5 selon la valence de l'élément Z ;
- m représente un nombre supérieur ou égal à 20 ;
- x représente un nombre de 6 à 12.

Les zéolites modifiées de formule (II) ou (III) peuvent comporter simultanément plusieurs des éléments représentés par X ou Z.

Les zéolites modifiées de type ZSM-5 et ZSM-11 de formule (II) ou (III) qui sont le plus généralement utilisées dans le procédé de l'invention sont celles pour lesquelles le symbole X ou le symbole Z représente plus particulièrement les éléments trivalents (Al, Ga, Fe, B, In, Cr et Sc) ou divalents (Co, Ni, Be, Zn et Cu) indiqués précédemment, respectivement seuls ou associés entre eux ou associés aux éléments pentavalents indiqués précédemment (Sb, As et V).

Le mode de préparation des zéolites de type ZSM-5 peut être celui qui est décrit dans le brevet américain n° 3 702 888, en remplaçant le composé d'aluminium par un composé inorganique de gallium, de fer, de bore, d'indium, de chrome, de scandium, de cobalt, de nickel, de beryllium, de zinc, de cuivre, d'antimoine, d'arsenic, de vanadium ou par un mélange de composés inorganiques de plusieurs de ces éléments.

On peut procéder en préparant une solution contenant de l'hydroxyde de tétraalkylammonium, tel que le tétrapropylammonium par exemple ; de l'oxyde de sodium ; de l'oxyde de gallium, de fer, de bore, d'indium, de chrome, de scandium, de cobalt, de nickel, de beryllium, de zinc, de cuivre, d'antimoine, d'arsenic, de vanadium ou un mélange de plusieurs de ces oxydes ; de la silice et de l'eau ; la solution comporte des rapports d'oxydes compris dans les intervalles suivants :

- $OH^-/SiO_2$ : de 0,07 à 10,0 ;
- tétraalkylammonium/tétraalkylammonium + $Na^+$ : de 0,2 à 0,95 ;

- $H_2O/OH^-$ : de 10 à 300 ;
- $SiO_2/X_2O_n$ supérieur ou égal à 20.

On peut utiliser un excès d'hydroxyde de tétraalkylammonium, ce qui augmente le rapport $OH^-/SiO_2$ indiqué précédemment. Mais l'excès d'hydroxyde ne participe pas à la réaction.

Le traitement pour l'obtention de cristaux de zéolites consiste généralement à chauffer le mélange précédent à une température de 100°C à 200°C pendant une durée de quelques heures à 60 jours sous pression autogène.

De préférence, les conditions de la réaction sont d'une dizaine d'heures à une dizaine de jours à une température de 150°C à 200°C. Ensuite les cristaux sont séparés du liquide par refroidissement à une température ambiante, filtration et lavage à l'eau.

La zéolite est ensuite séchée vers 100°C, en général pendant plusieurs heures. La préparation des zéolites de type ZSM-5 peut être effectuée en utilisant des composés qui génèrent les oxydes nécessaires.

Ainsi on peut mettre en oeuvre les nitrates de gallium, de fer, de bore, d'indium, de chrome, de scandium, de cobalt, de nickel, de beryllium, de zinc, de cuivre, d'antimoine, d'arsenic, de vanadium, le silicate de sodium, les gels de silice, l'acide silicique, l'hydroxyde de sodium ou des mélanges de ces différents composés avec les oxydes correspondants.

Le mode de préparation des zéolites de types ZSM-11 peut être celui qui est décrit dans le brevet américain n° 3 709 979 en remplaçant le cas échéant le composé d'aluminium par un composé inorganique de gallium, de fer, de bore, d'indium, de chrome, de scandium, de cobalt, de nickel, de beryllium, de zinc, de cuivre, d'antimoine, d'arsenic, de vanadium ou par un mélange de composés inorganiques de plusieurs de ces éléments.

On peut ainsi procéder comme décrit précédemment pour les zéolites de type ZSM-5 en préparant une solution contenant de l'oxyde de tétraalkylammonium ; de l'oxyde de sodium ; de l'oxyde d'aluminium, de gallium, de fer, de bore, d'indium, de chrome, de scandium, de cobalt, de nickel, de beryllium, de zinc, de cuivre, d'antimoine, d'arsenic, de vanadium ou un mélange de plusieurs de ces oxydes ; de la silice et de l'eau, les rapports d'oxydes de cette solution étant compris dans les intervalles suivants :

- $Na_2O/SiO_2$ : de 0,05 à 0,7 ;
- oxyde de tétraalkylammonium/$SiO_2$ : de 0,02 à 0,20 ;
- $H_2O/Na_2O$ : de 50 à 800 ;
- $SiO_2/Z_2O_n$ supérieur ou égal à 20.

Par zéolite modifiée, on entend dans le présent texte les zéolites de type ZSM-5 et ZSM-11 telles que définies précédemment, dont au moins une partie des cations M est constituée par l'hydrogène et de préférence la majorité ou la totalité des cations M est constituée par l'hydrogène.

Cela peut être obtenu en traitant les zéolites de type ZSM-5 et ZSM-11, dans la formule desquelles les cations M sont uniquement des métaux alcalins ou des ions tétraalkylammonium, par un acide organique ou inorganique.

A titre d'exemples d'acides inorganiques que l'on peut utiliser pour acidifier les zéolites, on peut citer l'acide chlorhydrique, l'acide sulfurique, l'acide nitrique, l'acide perchlorique et l'acide phosphorique.

A titre d'exemples d'acides organiques que l'on peut utiliser pour acidifier les zéolites, on peut citer les acides halosulfoniques comme l'acide chlorosulfonique, l'acide fluorosulfonique, les acides halométhanesulfoniques comme l'acide trifluorométhanesulfonique, les acides halocarboxyliques comme l'acide trichloroacétique, l'acide dichloroacétique, l'acide monochloroacétique, l'acide trifluoroacétique, l'acide difluoroacétique, l'acide monofluoroacétique, l'acide monobromoacétique.

Selon un mode de mise en oeuvre généralement utilisé, l'acidification de la zéolite de type ZSM-5 ou ZSM-11 se fait par passage de 10 $cm^3$ à 100 $cm^3$ d'acide, présentant une normalité de 0,1 N à 2 N, par gramme de zéolite.

Un tel passage peut être réalisé en une seule étape ou préférentiellement en plusieurs étapes successives.

Dans les formules (II) et (III) des zéolites de type ZSM-5 et ZSM-11 modifiées, le cation M qui éventuellement ne représente pas l'hydrogène est de préférence le sodium.

L'obtention des zéolites de types ZSM-5 et ZSM-11 modifiées peut également se faire par échange de tout ou partie des métaux alcalins à l'aide d'une solution de sel inorganique d'ammonium, tel que par exemple le nitrate d'ammonium, le fluorure d'ammonium, le chlorure d'ammonium, suivie d'un traitement thermique de quelques heures à des températures de 300°C à 800°C, dans un courant d'air sec afin de créer des sites $H^+$.

Dans le cadre de la présente invention, on préfère les zéolites de types ZSM-5 de formule (II) et de type ZSM-11 de formule (III) dans lesquelles le rapport $SiO_2/X_2O_n$ ou $SiO_2/Z_2O_n$ est compris entre 20 et 500.

Plus préférentiellement ce rapport $SiO_2/X_2O_n$ ou $SiO_2/Z_2O_n$ dans les formules (II) et (III) est compris entre 40 et 300.

Il est favorable de procéder à un traitement des zéolites de type ZSM-5 et ZSM-11 modifiées, afin d'améliorer leur activité catalytique dans la réaction d'hydroxylation des composés aromatiques de formule (I), à l'aide du protoxyde d'azote.

Cette activation peut notamment consister à effectuer un traitement thermique des zéolites modifiées, à une température de 300 °C à 800 °C et de préférence de 400 °C à 700 °C, pendant quelques heures, dans un courant de gaz inerte sec tel que l'azote. Eventuellement le traitement thermique peut être poursuivi pendant quelques heures à une température comprise dans les zones indiquées précédemment, mais sous courant d'air sec.

Les zéolites modifiées de type ZSM-5 et ZSM-11 peuvent être utilisées sous différentes formes dans le procédé de l'invention : poudre, notamment dans les essais de laboratoire, ou produits mis en forme tels que granulés (par exemple des cylindres ou des billes), billes, pastilles ou monolithes (blocs en forme de nid d'abeilles) qui sont obtenus par extrusion, moulage, compactage ou tout autre type de procédé connu.

En pratique sur le plan industriel, ce sont les formes de granulés, de billes et de monolithes qui présentent le plus d'avantages, tant sur le plan de l'efficacité que sur le plan de la commodité de la manipulation.

Le procédé de l'invention étant réalisé en phase vapeur, le problème de la récupération du catalyseur est ainsi résolu.

Parmi les composés de formule (I) on peut citer plus particulièrement le fluorobenzène, le phénol, le benzène, le toluène, l'orthocrésol, le métacrésol, le paracrésol, l'anisole, le bromobenzène, le chlorobenzène, l'orthochlorophénol, le parachlorophénol.

Le phénol, le fluorobenzène et le benzène sont des composés de formule (I) tout particulièrement intéressants à hydroxyler selon le procédé de l'invention.

Le protoxyde d'azote est utilisé pur ou en mélange avec un gaz inerte ne contenant pas d'oxygène tel que l'azote.

Le composé de formule (I) est de préférence introduit en mélange avec le protoxyde d'azote selon un rapport molaire du protoxyde d'azote par rapport au composé de formule (I) compris entre 1 et 10.

Selon un procédé de mise en oeuvre préférentiel, on vaporise le composé (I), on le mélange avec le protoxyde d'azote selon les proportions préalablement définies et on le fait circuler sur la zéolites de formule (II) ou (III). La réaction se déroule de préférence à une température comprise entre 300 et 500 °C.

Les gaz issus de la réaction contenant le cas échant un mélange d'isomères, sont condensés et séparés par toute technique connue de l'Homme de l'art.

Lorsqu'il est appliqué au phénol, le procédé de l'invention est particulièrement intéressant car il permet d'obtenir les 3 dihydroxybenzènes, pyrocatéchol, hydroquinone et résorcinol, alors que les procédés connus d'hydroxylation du phénol conduisent pratiquement uniquement à un mélange pyrocatéchol-hydroquinone. Le procédé fournit donc une voie originale d'accès au résorcinol.

Les exemples qui suivent illustrent l'invention.

EXEMPLE 1 - Préparation d'une zéolite de type ZSM-5 au gallium

Une solution de 5,94 g de nitrate de gallium hydraté ($Ga(NO_3)_3$, 6 $H_2O$) et de 16 g de $H_2SO_4$ (à 96 %) dans 200 g d'eau, est coulée dans une solution de 200 g de silicate de sodium ($SiO_2/Na_2O$ = 3,22) dans 200 g d'eau.

Au gel ainsi obtenu sont ajoutés 24 g de bromure de tétrapropylammonium dans 100 g d'eau.

On agite plusieurs minutes, puis le mélange est placé dans un autoclave agité de 1 litre, sous pression autogène, pendant 4 jours à 170 °C.

Le solide blanc ainsi obtenu est filtré, lavé à l'eau et séché à 100 °C.

Une analyse de la poudre par diffraction X confirme la formation de zéolite de type ZSM-5 au gallium.

Le rapport molaire $SiO_2/Ga_2O_3$ est de 80.

L'activation de cette zéolite au gallium consiste à la traiter thermiquement à 550 °C, pendant 18 heures sous courant d'azote sec, puis pendant 5 heures à 550 °C sous courant d'air sec.

Après refroidissement, la zéolite est échangée avec une solution 1 M de $NH_4NO_3$ et traitée thermiquement pendant 10 heures dans un courant d'air sec, afin de former les sites $H^+$.

EXEMPLE 2 - Préparation d'une zéolite de type ZSM-5 au fer

Une solution de 50 g de métasilicate de sodium ($Na_2SiO_3$, 5 $H_2O$) dans 50 g d'eau est coulée dans une solution contenant 2,1 g de nitrate ferrique ($Fe(NO_3)_3$, 9 $H_2O$) dans 50 g d'eau.

Le pH est ajusté à une valeur fortement acide à l'aide de 5,0 g de $H_2SO_4$ à 96 %.

Au gel obtenu, de couleur citron pâle, sont ajoutés 6,5 g de bromure de tétrapropylammonium dans 10 g d'eau.

Après agitation vigoureuse, le mélange est placé dans un autoclave en acier inoxydable, fermé et chauffé sous pression autogène et sous agitation pendant 5 jours à 170 °C.

Le solide blanc obtenu est filtré, lavé à l'eau et séché à 100 °C.

Une analyse de la poudre par diffraction X confirme la formation de zéolite de type ZSM-5 au fer.

Le rapport molaire $SiO_2/Fe_2O_3$ est de 90.

On prépare selon le même mode opératoire des zéolites au fer ayant respectivement des rapports molaires $SiO_2/Fe_2O_3$ de 120 et 150, en modifiant la proportion métasilicate de sodium/nitrate ferrique et en ajustant la teneur en hydroxyde.

L'activation de ces zéolites au fer se fait de la manière décrite dans l'exemple 1 pour former les sites $H^+$.

EXEMPLE 3 - Préparation d'une zéolite de type ZSM-5 au cobalt

On prépare dans un premier temps une solution A de 0,27 g de $Co(NO_3)_2$, 6 $H_2O$ et O,6 g de $H_2SO_4$ dans 15 g d'eau.

On prépare ensuite une solution B de 10 g de silice (commercialisée sous la marque Q-Brand) dans 10 g d'eau, puis une solution C de 1 g de bromure de tétrapropylammonium dans 10 g d'eau.

On mélange tout d'abord les solutions A et B rapidement et on agite jusqu'à obtention d'un gel.

On ajoute alors à ce gel la solution C sous agitation douce.

Le gel ainsi obtenu est placé dans un autoclave agité pendant 3 jours à 170 °C

Le solide blanc obtenu est filtré, lavé à l'eau et séché à 100 °C.

Une analyse de la poudre par diffraction X confirme la formation de zéolite de type ZSM-5 au cobalt.

Le rapport molaire $SiO_2$/2 CoO est de 80.

L'activation de cette zéolite au cobalt se fait de la manière décrite dans l'exemple 1 pour former les sites $H^+$.

EXEMPLES 4 - Préparation d'une zéolite de type ZSM-11 à l'aluminium

On prépare une solution A contenant 55 g de silicate de sodium ($SiO_2/Na_2O$ = 3,22) et 1,3 g de diamino-1,8 octane dans 38 g d'eau.

On prépare une solution B contenant 0,7 g d'aluminate de sodium dans 40 g d'eau.

On mélange les deux solutions A et B.

Le pH du gel ainsi obtenu est ajusté à 11 à l'aide d'acide sulfurique.

Le gel est placé dans un autoclave, agité pendant 3 jours à 220 °C.

Le solide blanc obtenu est filtré, lavé à l'eau et séché à 100 °C.

Une analyse de la poudre par diffraction X confirme la formation de zéolite de type ZSM-11 à l'aluminium.

Le rapport molaire $SiO_2/Al_2O_3$ est de 40.

L'activation de cette zéolite à l'aluminium se fait de la manière décrite dans l'exemple 1 pour former les sites $H^+$.

EXEMPLES 5 à 10 - Hydroxylation du fluorobenzène en fluorophénols

Conditions expérimentales

- phase vapeur : en continu
- catalyseur : zéolite de type ZSM-5 ou ZSM-11 modifiée préparée dans les exemples 1, 2, 3 et 4 (voir tableau ci-après)
- température : 400 °C
- vélocité spatiale liquide heure (poids de substrat/poids de catalyseur/heure : 1,5 $h^{-1}$
- rapports molaires fluorobenzène/$N_2/N_2O$ : 2 / 5/ 8

2 $cm^3$ de zéolite modifiée (environ 1 g) en poudre, dispersé dans 4 $cm^3$ de quartz en grains (inférieurs à 0,8 mm) sont introduits dans un réacteur tubulaire en quartz de longueur 16 cm et diamètre intérieur 1,8 cm.

On ajoute ensuite dans le réacteur un lit de 10 $cm^3$ de billes de verre permettant d'homogénéiser le mélange gazeux.

Le réacteur ainsi chargé est conditionné 2 heures à 400 °C sous azote dans un four tubulaire.

La réaction est effectuée en continu, en introduisant 1,5 $cm^3/h$ de fluorobenzène, 1,44 l/h de $N_2O$ et 0,9 l/h d'azote.

Les résultats sont indiqués dans le tableau ci-après.

| Exemples | Zéolite modifiée | Rapport molaire $SiO_2/X_2O_n$ ou $SiO_2/Z_2O_n$ | TT % du fluorobenzène | RT % en fluorophénols |
|---|---|---|---|---|
| Exemple 5 | ZSM-5 Ga | 80 | 4,1 | 75 |
| Exemple 6 | ZSM-5 Co | 80 | 1,3 | 89 |
| Exemple 7 | ZSM-5 Fe | 90 | 16,0 | 90 |
| Exemple 8 | ZSM-5 Fe | 120 | 9,8 | 93 |
| Exemple 9 | ZSM-5 Fe | 150 | 8,4 | 94 |
| Exemple 10 | ZSM-11 Al | 40 | 1,2 | 69 |

TT % = taux de transformation
RT % = rendement par rapport au fluorobenzène transformé

## Revendications

**1.** Procédé d'hydroxylation d'un composé aromatique de formule (I) :

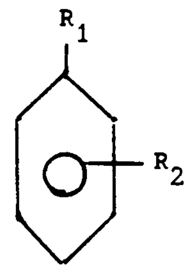

(I)

dans laquelle :

- $R_1$ représente :
  - . un groupement OH,
  - . un atome de brome,
  - . un atome de chlore,
  - . un atome de fluor,
  - . un atome d'hydrogène,
  - . un radical alkyle, linéaire ou ramifié, ayant 1 à 6 atomes de carbone,
  - . un radical alcoxy, linéaire ou ramifié, ayant 1 à 6 atomes de carbone,
- $R_2$ représente :
  - . un atome d'hydrogène,
  - . un radical alkyle, linéaire ou ramifié, ayant 1 à 6 atomes de carbone,
  - . un radical alcoxy, linéaire ou ramifié, ayant 1 à 6 atomes de carbone,

caractérisé en ce que l'on met en présence, à une température de 250 °C à 500 °C, le composé aromatique de formule (I), du protoxyde d'azote et une zéolite modifiée choisie parmi :

a) les zéolites modifiées de type ZSM-5 de formule générale (II) exprimée en termes de rapports d'oxydes :

$M_2O, X_2O_n, m\ SiO_2, p\ H_2O$ (II)

dans laquelle :

- M représente un cation choisi parmi l'hydrogène et les métaux alcalins, M étant au moins en partie un atome d'hydrogène ;
- X représente Ga, Fe, B, In, Cr, Sc, Co, Ni, Be, Zn, Cu, Sb, As, V ;
- n représente 2, 3 ou 5 selon la valence de l'élément X ;
- m représente un nombre supérieur ou égal à 20 ; - p représente un nombre de 0 à 40 ;

b) les zéolites modifiées de type ZSM-11 de formule générale (III), exprimée en termes de rapports d'oxydes :

$M_2O, Z_2O_n, m\ SiO_2, x\ H_2O$ (III)

dans laquelle :

- M représente un cation choisi parmi l'hydrogène et les métaux alcalins, M étant au moins en partie un atome d'hydrogène ;
- Z représente Al, Ga, Fe, B, In, Cr, Sc, Co, Ni, Be, Zn, Cu, Sb, As, V ;
- n représente 2, 3 ou 5 selon la valence de l'élément Z ;
- m représente un nombre supérieur ou égal à 20 ;
- x représente un nombre de 6 à 12.

**2.** Procédé selon la revendication 1, caractérisé en ce que les zéolites modifiées de type ZSM-5 et ZSM-11 qui sont utilisées dans le procédé de l'invention sont celles pour lesquelles le symbole X ou le symbole Z représente les éléments trivalents (Al, Ga, Fe, B, In, Cr et Sc) ou divalents (Co, Ni, Be, Zn et Cu) indiqués dans la revendication 1, respectivement seuls ou associés entre eux ou associés aux

éléments Sb, As et/ou V.

**3.** Procédé selon l'une des revendications 1 ou 2, caractérisé en ce que les zéolites modifiées de type ZSM-5 et ZSM-11 sont obtenues en traitant les zéolites de type ZSM-5 et ZSM-11, dans la formule desquelles les cations M sont uniquement des métaux alcalins ou des ions tétraalkylammonium, par un acide organique ou inorganique.

**4.** Procédé selon la revendication 3, caractérisé en ce que l'on utilise un acide inorganique choisi parmi l'acide chlorhydrique, l'acide sulfurique, l'acide nitrique, l'acide perchlorique et l'acide phosphorique.

**5.** Procédé selon la revendication 3, caractérisé en ce que l'on utilise un acide organique choisi parmi les acides halosulfoniques comme l'acide chlorosulfonique, l'acide fluorosulfonique, les acides halométhanesulfoniques comme l'acide trifluorométhanesulfonique, les acides halocarboxyliques comme l'acide trichloroacétique, l'acide dichloroacétique, l'acide monochloroacétique, l'acide trifluoroacétique, l'acide difluoroacétique, l'acide monofluoroacétique, l'acide monobromoacétique.

**6.** Procédé selon l'une des revendications 3 à 5, caractérisé en ce que l'acidification de la zéolite de type ZSM-5 ou ZSM-11 se fait par passage de 10 $cm^3$ à 100 $cm^3$ d'acide, présentant une normalité de 0,1 N à 2 N, par gramme de zéolite.

**7.** Procédé selon la revendication 6, caractérisé en ce que l'acidification de la zéolite de type ZSM-5 ou ZSM-11 est réalisée en une seule étape ou préférentiellement en plusieurs étapes successives.

**8.** Procédé selon l'une des revendications 1 ou 2, caractérisé en ce que dans les formules (II) et (III) des zéolites de type ZSM-5 et ZSM-11 modifiées, le cation M qui éventuellement ne représente pas l'hydrogène est de préférence le sodium.

**9.** Procédé selon l'une des revendications 1 ou 2, caractérisé en ce que les zéolites modifiées de type ZSM-5 et ZSM-11 sont obtenues par échange de tout ou partie des métaux alcalins à l'aide d'une solution d'un sel inorganique d'ammonium, suivie d'un traitement thermique de quelques heures à des températures de 300 °C à 800 °C, dans un courant d'air sec afin de créer des sites $H^+$.

**10.** Procédé selon l'une des revendications 1 à 9, caractérisé en ce que l'on utilise les zéolites de types ZSM-5 de formule (II) et de type ZSM-11 de formule (III) dans lesquelles le rapport $SiO_2/X_2O_n$ ou $SiO_2/Z_2O_n$ est compris entre 20 et 500.

**11.** Procédé selon l'une des revendications 1 à 10, caractérisé en ce que l'on utilise les zéolites de types ZSM-5 de formule (II) et de type ZSM-11 de formule (III) dans lesquelles le rapport $SiO_2/X_2O_n$ ou $SiO_2/Z_2O_n$ est compris entre 40 et 300.

**12.** Procédé selon l'une des revendications 1 à 11, caractérisé en ce que l'on procède à un traitement des zéolites de type ZSM-5 et ZSM-11 modifiées, à une température de 300 °C à 800 °C et de préférence de 400 °C à 700 °C, pendant quelques heures, dans un courant de gaz inerte sec tel que l'azote.

**13.** Procédé selon la revendication 12, caractérisé en ce que le traitement thermique est poursuivi pendant quelques heures à une température comprise dans les zones indiquées précédemment, mais sous courant d'air sec.

**14.** Procédé selon l'une des revendications 1 à 13, caractérisé en ce que le composé de formule (I) est choisi parmi le fluorobenzène, le phénol, le benzène, le toluène, l'orthocrésol, le métacrésol, le paracrésol, l'anisole, le bromobenzène, le chlorobenzène, l'orthochlorophénol, le parachlorophénol.

**15.** Procédé selon l'une des revendications 1 à 13, caractérisé en ce que le composé de formule (I) est choisi parmi le fluorobenzène, le phénol et le benzène.

**16.** Procédé selon l'une des revendications 1 à 15, caractérisé en ce que le composé de formule (I) est introduit en mélange avec le protoxyde d'azote selon un rapport molaire du protoxyde d'azote par rapport au composé de formule (I) compris entre 1 et 10.

## Claims

**1.** Process for the hydroxylation of an aromatic compound of formula (I):

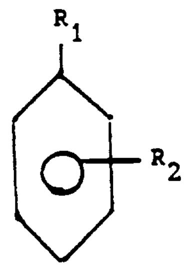

(I)

in which:

- $R_1$ represents:
  - an OH group,
  - a bromine atom,
  - a chlorine atom,
  - a fluorine atom,
  - a hydrogen atom,
  - a linear or branched alkyl radical having 1 to 6 carbon atoms,
  - a linear or branched alkoxy radical having 1 to 6 carbon atoms,
- $R_2$ represents:
  - a hydrogen atom,
  - a linear or branched alkyl radical having 1 to 6 carbon atoms,
  - a linear or branched alkoxy radical having 1 to 6 carbon atoms,

characterized in that the aromatic compound of formula (I) is brought together at a temperature from 250 ° C to 500 ° C with nitrous oxide and a modified zeolite chosen from:

a) modified zeolites of ZSM-5 type of general formula (II), expressed in terms of oxide ratios:

$M_2O.\ X_2O_n.\ m\ SiO_2.\ p\ H_2O$ (II)

in which:

- M represents a cation chosen from hydrogen and the alkali metals, M being, at least in part, a hydrogen atom;
- X represents Ga, Fe, B, In, Cr, Sc, Co, Ni, Be, Zn, Cu, Sb, As, V;
- n represents 2, 3 or 5 depending on the valence of the element X;
- m represents a number greater than or equal to 20;
- p represents a number from 0 to 40;

b) modified zeolites of ZSM-11 type of general formula (III), expressed in terms of oxide ratios:

$M_2O.\ Z_2O_n.\ m\ SiO_2.\ x\ H_2O$ (III)

in which:

- M represents a cation chosen from hydrogen and the alkali metals, M being, at least in part, a hydrogen atom;
- Z represents Al, Ga, Fe, B, In, Cr, Sc, Co, Ni, Be, Zn, Cu, Sb, As, V;
- n represents 2, 3 or 5 depending on the valence of the element Z;
- m represents a number greater than or equal to 20;
- x represents a number from 6 to 12.

**2.** Process according to claim 1, characterized in that the modified zeolites of ZSM-5 and ZSM-11 type which are used in the process of the invention are those for which the symbol X or the symbol Z represents the trivalent elements (Al, Ga, Fe, B, In, Cr and Sc) or the bivalent elements (Co, Ni, Be, Zn and Cu) indicated in claim 1, alone or combined with themselves or combined with the elements Sb, As and/or V respectively.

**3.** Process according to either of claims 1 and 2, characterized in that the modified zeolites of ZSM-5 and ZSM-11 type are obtained by treating the zeolites of ZSM-5 and ZSM-11 type, in the formula for which the cations M are exclusively alkali metals or tetraalkylammonium ions, with an organic or inorganic acid.

**4.** Process according to claim 3, characterized in that an inorganic acid chosen from hydrochloric acid, sulphuric acid, nitric acid, perchloric acid and phosphoric acid is used.

**5.** Process according to claim 3, characterized in that an organic acid chosen from halosulphonic acids such as chlorosulphonic acid and fluorosulphonic acid, halomethanesulphonic acids such as trifluoromethanesulphonic acid and halocarboxylic acids such as trichloroacetic acid, dichloroacetic acid, monochloroacetic acid, trifluoroacetic acid, difluoroacetic acid, monofluoroacetic acid and monobromoacetic acid is used.

**6.** Process according to one of claims 3 to 5, characterized in that acidification of ZSM-5 or ZSM-11 type zeolite is carried out by passing through it from 10 $cm^3$ to 100 $cm^3$ of acid, having a normality from 0.1 N to 2 N, per gram of zeolite.

**7.** Process according to claim 6, characterized in that acidification of ZSM-5 or ZSM-11 type zeolite is carried out in a single step or, preferably, in several successive steps.

**8.** Process according to either of claims 1 and 2, characterized in that in the formulae (II) and (III) for the modified ZSM-5 and ZSM-11 type zeolites the cation M, which optionally does not represent hydrogen, is preferably sodium.

**9.** Process according to either of claims 1 and 2, characterized in that the modified zeolites of ZSM-5 and ZSM-11 type are obtained by exchange of all or part of the alkali metals with the aid of a solution of an inorganic ammonium salt, followed by a thermal treatment for a few hours at temperatures from 300 °C to 800 °C in a stream of dry air, in order to create $H^+$ sites.

**10.** Process according to one of claims 1 to 9, characterized in that ZSM-5 type zeolites of formula (II) and ZSM-11 type zeolites of formula (III) in which formulae the $SiO_2/X_2O_n$ or $SiO_2/Z_2O_n$ ratio is between 20 and 500 are used.

**11.** Process according to one of claims 1 to 10, characterized in that ZSM-5 type zeolites of formula (II) and ZSM-11 type zeolites of formula (III) in which formulae the $SiO_2/X_2O_n$ or $SiO_2/Z_2O_n$ ratio is between 40 and 300 are used.

**12.** Process according to one of claims 1 to 11, characterized in that the modified ZSM-5 and ZSM-11 type zeolites are treated at a temperature from 300 °C to 800 °C, and preferably from 400 °C to 700 °C, for a few hours in a stream of a dry inert gas such as nitrogen.

**13.** Process according to claim 12, characterized in that the thermal treatment is continued for a few hours at a temperature within the regions indicated above, but under a stream of dry air.

**14.** Process according to one of claims 1 to 13, characterized in that the compound of formula (I) is chosen from fluorobenzene, phenol, benzene, toluene, ortho-cresol, meta-cresol, para-cresol, anisole, bromobenzene, chlorobenzene, ortho-chlorophenol and parachlorophenol.

**15.** Process according to one of claims 1 to 13, characterized in that the compound of formula (I) is chosen from fluorobenzene, phenol and benzene.

**16.** Process according to one of claims 1 to 15, characterized in that the compound of formula (I) is introduced as a mixture with nitrous oxide according to a molar ratio of nitrous oxide relative to the compound of formula (I) of between 1 and 10.

## Patentansprüche

**1.** Verfahren zur Hydroxilierung einer aromatischen Verbindung der Formel (I):

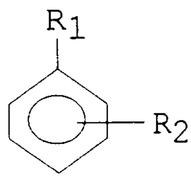

(I)

wobei:

- $R_1$ darstellt:
  - eine OH-Gruppe
  - ein Bromatom
  - ein Chloratom
  - ein Fluoratom
  - ein Wasserstoffatom
  - einen linearen oder verzweigten Alkylrest mit 1 bis 6 Kohlenstoffatomen
  - einen linearen oder verzweigten Alkoxyrest mit 1 bis 6 Kohlenstoffatomen
- $R_2$ darstellt:
  - ein Wasserstoffatom
  - einen linearen oder verzweigten Alkylrest mit 1 bis 6 Kohlenstoffatomen
  - einen linearen oder verzweigten Alkoxyrest mit 1 bis 6 Kohlenstoffatomen

dadurch gekennzeichnet, daß die aromatische Verbindung der Formel (I) bei einer Temperatur zwischen 250 °C und 500 °C mit Distickstoffmonoxid und einem modifiziertem Zeolithen zusammengegeben wird; der modifizierte Zeolith wird gewählt aus:

a) den modifizierten Zeolithen des Typs ZSM-5 der allgemeinen Formel (II), ausgedrückt in Verhältnisse der Oxide:

$M_2O, X_2O_n, m\ SiO_2, p\ H_2O$ (II)

wobei:

- M ein Kation darstellt, das aus Wasserstoff und den Alkalimetallen ausgewählt ist, wobei M wenigstens zum Teil ein Wasserstoffatom ist;
- X für Ga, Fe, B, In, Cr, Sc, Co, Ni, Be, Zn, Cu, Sb, As oder V steht;
- n 2, 3 oder 5 entspricht, abhängig von der Wertigkeit des Elementes X;
- m eine Zahl größer oder gleich 20 ist;
- p eine Zahl zwischen 0 und 40 ist;

b) den modifizierten Zeolithen des Typs ZSM-11 der allgemeinen Formel (III), ausgedrückt in Verhältnisse der Oxide:

$M_2O, Z_2O_n, m\ SiO_2, x\ H_2O$ (III)

wobei:

- M ein Kation darstellt, das aus Wasserstoff und den Alkalimetallen ausgewählt ist, wobei M wenigstens zum Teil ein Wasserstoffatom ist;
- Z für Al, Ga, Fe, B, In, Cr, Sc, Co, Ni, Be, Zn, Cu, Sb, As oder V steht;
- n 2, 3 oder 5 entspricht, abhängig von der Wertigkeit des Elementes Z;
- m eine Zahl größer oder gleich 20 ist;
- p eine Zahl zwischen 6 und 12 ist.

**2.** Verfahren nach Anspruch 1, dadurch gekennzeichnet, daß die in dem erfindungsgemäßen Verfahren verwendeten modifizierten Zeolithe des Typs ZSM-5 und ZSM-11 derart sind, daß das Symbol X oder das Symbol Z dreiwertige Elemente (Al, Ga, Fe, B, In, Cr und Sc) oder zweiwertige Elemente (Co, Ni, Be, Zn und Cu), wie in Anspruch 1 aufgeführt, angeben, wobei die Elemente entweder einzeln vorliegen oder mit sich selbst oder mit den Elementen Sb, As und/oder V assoziiert sind.

3. Verfahren nach einem der Ansprüche 1 oder 2, dadurch gekennzeichnet, daß die modifizierten Zeolithe des Typs ZSM-5 und ZSM-11 durch Behandlung mit einer organischen oder anorganischen Säure erhalten werden, wobei in der Formel der Zeolithe des Typs ZSM-5 und ZSM-11 die Kationen M einheitlich Alkalimetall- oder Tetraalkylammoniumionen sind.

4. Verfahren nach Anspruch 3, dadurch gekennzeichnet, daß die verwendete anorganische Säure aus Salzsäure, Schwefelsäure, Salpetersäure, Perchlorsäure und Phosphorsäure ausgewählt ist.

5. Verfahren nach Anspruch 3, dadurch gekennzeichnet, daß die verwendete organische Säure aus Halogensulfonsäuren, wie z.B. Chlorsulfonsäure oder Fluorsulfonsäure, Halogenmethansulfonsäuren, wie z.B. Trifluormethansulfonsäure, Halogencarbonsäuren, wie z.B. Trichloressigsäure, Dichloressigsäure, Monochloressigsäure, Trifluoressigsäure, Difluoressigsäure, Monofluoressigsäure oder Monobromessigsäure, ausgewählt ist.

6. Verfahren nach einem der Ansprüche 3 bis 5, dadurch gekennzeichnet, daß die Acidifizierung der Zeolithe des Typs ZSM-5 oder ZSM-11 erreicht wird, indem 10 $cm^3$ bis 100 $cm^3$ Säure mit einer Normalität von 0,1 N bis 2 N pro Gramm Zeolith durch den Zeolithen geleitet werden.

7. Verfahren nach Anspruch 6, dadurch gekennzeichnet, daß die Acidifizierung der Zeolithe des Typs ZSM-5 oder ZSM-11 in einem einzelnen Schritt oder vorzugsweise in mehreren aufeinanderfolgenden Schritten durchgeführt wird.

8. Verfahren nach einem der Ansprüche 1 oder 2, dadurch gekennzeichnet, daß in den Formeln (II) und (III) der zu modifizierenden Zeolithe des Typs ZSM-5 und ZSM-11 das Kation M, das gegebenenfalls kein Wasserstoffatom ist, vorzugsweise Natrium ist.

9. Verfahren nach einem der Ansprüche 1 oder 2, dadurch gekennzeichnet, daß die modifizierten Zeolithe des Typs ZSM-5 und ZSM-11 dadurch erhalten werden, daß die Alkalimetalle vollständig oder zum Teil mit Hilfe einer Lösung eines anorganischen Ammoniumsalzes ausgetauscht werden, gefolgt von einer Wärmebehandlung über mehrere Stunden bei Temperaturen von 300 °C bis 800 °C unter einem trokkenen Luftstrom, um $H^+$-Stellen zu erzeugen.

10. Verfahren nach einem der Ansprüche 1 bis 9, dadurch gekennzeichnet, daß Zeolithe des Typs ZSM-5 der Formel (II) und des Typs ZSM-11 der Formel (III) verwendet werden, wobei das Verhältnis von $SiO_2/X_2O_n$ oder $SiO_2/Z_2O_n$ zwischen 20 und 500 liegt.

11. Verfahren nach einem der Ansprüche 1 bis 10, dadurch gekennzeichnet, daß Zeolithe des Typs ZSM-5 der Formel (II) und des Typs ZSM-11 der Formel (III) verwendet werden, wobei das Verhältnis $SiO_2/X_2O_n$ oder $SiO_2/Z_2O_n$ zwischen 40 und 300 liegt.

12. Verfahren nach einem der Ansprüche 1 bis 11, dadurch gekennzeichnet, daß die Behandlung der zu modifizierenden Zeolithe des Typs ZSM-5 und ZSM-11 bei einer Temperatur zwischen 300 °C und 800 °C, vorzugsweise zwischen 400 °C und 700 °C über mehrere Stunden unter einem Inertgasstrom, wie zum Beispiel einem Stickstoffstrom, durchgeführt wird.

13. Verfahren nach Anspruch 12, dadurch gekennzeichnet, daß die Wärmebehandlung über mehrere Stunden bei einer Temperatur in den zuvor angegebenen Bereichen durchgeführt wird, aber unter einem trokkenem Luftstrom.

14. Verfahren nach einem der Ansprüche 1 bis 13, dadurch gekennzeichnet, daß die Verbindung der Formel (I) aus Fluorbenzol, Phenol, Benzol, Toluol, Orthokresol, Metakresol, Parakresol, Anisol, Brombenzol, Chlorbenzol, Orthochlorphenol und Parachlorphenol ausgewählt ist.

15. Verfahren nach einem der Ansprüche 1 bis 13, dadurch gekennzeichnet, daß die Verbindung der Formel (I) aus Fluorbenzol, Phenol und Benzol ausgewählt ist.

16. Verfahren nach einem der Ansprüche 1 bis 15, dadurch gekennzeichnet, daß die Verbindung der Formel (I) mit Distickstoffmonoxid in einem Stoffmengenverhältnis von Distickstoffmonoxid zu der

Verbindung der Formel (I) zwischen 1 und 10 vermischt wird.